# EUROPEAN PATENT SPECIFICATION

(11) **EP 3 920 902 B1**
(45) Date of publication and mention of the grant of the patent: **05.08.2026**
(21) Application number: 20702843.2
(22) Date of filing: 10.02.2020
(51) Int. Cl.: A61K 31/19, A61K 45/06, A61P 31/04, A01K 61/13, A23K 50/80, A61K 31/20, A23K 20/174, A23K 20/20

(54) **COMPOSITION FOR USE IN THE TREATMENT OF PISCIRICKETTSIOSIS**
ZUSAMMENSETZUNG ZUR BEHANDLUNG VON PISCIRICKETTSIOSIS
COMPOSITION DESTINÉE À ÊTRE UTILISÉE DANS LE TRAITEMENT DE LA PISCIRICKETTSIOSE

(30) Priority: 08.02.2019 BE 201905082
(43) Date of publication of application: 15.12.2021
(73) Proprietor: Nutrition Sciences N.V., 9031 Drongen (BE)
(72) Inventor: BRUGGEMAN, Geert, 8200 Brugge (BE); BRUGGER, Roland, 9000 Ghent (BE)
(74) Representative: Brantsandpatents bv
(86) International application number: PCT/EP2020/053269
(87) International publication number: WO 2020/161349

(56) References cited:
- EP-A1- 1 123 701
- WO-A1-2011/119047
- WO-A1-2015/052672
- WO-A2-2004/091307
- CN-A- 108 289 478
- NO-A1- 20 171 171
- DATABASE WPI Week 199228, Derwent World Patents Index; AN 1992-231738, XP002793573
- M ROZAS ET AL: "Piscirickettsiosis and Piscirickettsia salmonis in fish: a review", JOURNAL OF FISH DISEASES, vol. 37, no. 3, 26 March 2014 (2014-03-26), pages 163 - 188, XP055144904, ISSN: 0140-7775, DOI: 10.1111/jfd.12211

## Description

### TECHNICAL FIELD

This invention relates generally to the field of obligate intracellular bacteria, and in particular to agents of rickettsia type diseases, specifically Piscirickettsia salmonis in aquatic poikilotherms.

### BACKGROUND

Piscirickettsia salmonis was the first member of the order Rickettsiales to be isolated from an aquatic poikilothermic organism. P. salmonis is the etiological agent of salmonid rickettsial septicemia or piscirickettsiosis, and is an economically significant pathogen of salmonids that is responsible for extensive mortalities in the cold water aquaculture industry. P. salmonis, a gram-negative obligate intracellular bacterium, was first observed in 1989 in a diseased, moribund Coho salmon from a saltwater net pen site on the coast of Chile. It is now known that P. salmonis is geographically more widespread than was initially suspected. P. salmonis has been observed to infect a wide range of salmonid species and causes a systemic infection that targets the kidney, liver, spleen, heart, brain, intestine, ovary, and gills of salmonids.

Piscirickettsiosis is the main health challenge in the cold water aquaculture sector and the disease has a huge economic impact on the sector. Indeed, outbreaks of the disease in the past have resulted in losses worth approximately up to hundreds of millions of US dollars in the sector.

Currently, the sector has focused its control strategies for the disease on antimicrobial therapies and vaccines. Piscirickettsiosis has evolved over time where each new outbreak is increasingly insidious and refractory to treatments, and each has shown increased bacterial virulence, clinical and pathological severity and variable presentation under similar conditions of species, age and management measures.

The use of antibiotics, both prophylactically and during early infection have been largely unsuccessful in stopping disease outbreaks. Similarly, commercial vaccines against P. salmonis have not proven to be of high efficacy.

JP283135B2 describes the use of an antimicrobial composition comprising a combination of a medium chain-fatty acid (MCFA), a monoglyceride and a diglyceride of the MCFA for the prevention of growth of a number of harmful bacteria. WO2015052672, EP1123701, WO2004091307 and DATABASE WPI week 199228 Thomson Scientific, London, GB also describe the use of feed products comprising MCFAs with antibacterial properties. However, they do not describe antimicrobial activity of this composition towards Piscirickettsia salmonis or towards any other bacteria that can cause piscirickettsiosis.

WO2011119047, NO20171171 and M. Rozas et al., 2014 describe various approaches to treatment piscirickettsiosis. However, these documents do not refer to compositions based on MCFAs.

The current approaches therefore do not satisfy the needs of the cold water aquaculture sector.

The present invention aims to provide an alternative for the treatment and prevention of piscirickettsiosis.

### SUMMARY OF THE INVENTION

The invention thereto aims to provide a composition for use in the treatment, suppression and /or prevention of piscirickettsiosis according to claim 1. More in particular, the invention provides a composition comprising one or more medium-chain fatty acids (MCFAs) and / or derivatives thereof. In a further aspect, the present invention provides a fish feed according to claim 13.

The inventors have found that a composition according to the invention is surprisingly effective for the prevention, suppression, and/or treatment of piscirickettsiosis, thus allowing to significantly increase both the productivity and the profitability of aquaculture. In addition, the current invention also provides a considerable contribution to the reduction of animal suffering and the improvement of animal welfare. Finally, the composition for use according to the invention also reduces the need for administering antibiotics to the animals during aquaculture thus helping to prevent the further upcoming spread of drug resistant pathogens.

### DETAILED DESCRIPTION OF THE INVENTION

The references to e methods of treatment by therapy in the description are to be interpreted as references to compositions of the present invention for use in those methods. The statements relating to non P. salmonis organisms and enhancing the zootechnical performance are not according to the invention and are present for illustration purpose only.

The present invention concerns a composition for use in the prevention, suppression and / or treatment of piscirickettsiosis in aquatic poikilotherms, characterized in that said composition comprises one or more medium-chain fatty acids (MCFAs) or derivatives thereof.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The recitation of numerical ranges by endpoints includes all numbers and fractions subsumed within that range, as well as the recited endpoints.

The expression "% by weight", "weight percent", "%wt" or "wt%", here and throughout the description unless otherwise defined, refers to the relative weight of the respective component based on the overall weight of the formulation.

The term "medium-chain fatty acid" or "MCFA" as used herein, refers to fatty acids with a medium-chain length, wherein the fatty acids may be saturated or unsaturated. In the present invention, the longest continuous chain of an MCFA can consist of 5 to 12 carbon atoms, for example, valeric acid (C5), caproic acid (C6), caprylic acid (C8), capric acid (C10) or lauric acid (C12). The term MCFA also refers to medium-chain fatty acids that are chemically modified, and to medium-chain fatty acids that are provided with side-chains, such as, without limitation, one or more alkyl groups, preferably C1-C10 alkyl groups. As described herein, the term "medium-chain fatty acid (MCFA) derivative" refers to a fatty acid chain of which the carboxyl group is reversibly converted to a different group, preferably, but without limitation, to an amide, salt, ester or glyceride such as, for example, a mono-, di- or tri-glyceride.

The term "piscirickettsiosis" as used herein, refers to an infectious disease affecting aquatic poikilothermic organisms caused by Piscirickettsia salmonis (P. salmonis), Piscirickettsia-like organisms (PLO) and/or fish rickettsia-like organisms (RLO). The term piscirickettsiosis as used herein, also refers to the disease salmonid rickettsial septicemia (SRS).

"Piscirickettsia-like organisms" (PLO) and "fish rickettsia-like organisms" (RLO), as used herein are organisms whose developmental stages are morphologically similar to that of organisms belonging to the family of Piscirickettsiaceae and/or to that of organisms belonging to the order of Rickettsiales and which cause disease in aquatic poikilothermic organisms resulting in symptoms similar to those caused by infection of salmonids with P. salmonis.

The term "aquatic poikilotherm" or "aquatic poikilothermic organism" as used herein, refers to aquatic animals whose internal temperature varies considerably, and most of the time, depends on the environment. Aquatic poikilotherms that can suffer from piscirickettsiosis include, but are not limited to salmonids, white seabass (*Atactoscion noblis*)*,* black seabass (*Dicentrarchus sp*.)*,* tilapia (*Oreochromis, Tilapia* and *Sarotherodon spp*.) and blue-eyed plecostomus (*Panaque suttoni*)*.*

The term "salmonid" refers to fish species belonging to the family of Salmonidae within the order of Salmoniformes. Salmonids include, but are not limited to, salmons, trouts, chars, freshwater whitefishes and graylings. Salmons include, but are not limited to, Atlantic salmon species such as Atlantic salmon (*Salmo salar*) and Pacific salmon species such as Chinook salmon (*Oncorhynchus tshawytscha*)*,* Chum salmon (*O*. *keta*)*,* Coho salmon (*O. kisutch*)*,* Masu salmon (*O*. *masou),* Pink salmon (*O. gorbuscha*) and Sockeye salmon (*O*. *nerka*)*.*

The term "Feed Conversion Ratio" or "FCR" describes the efficiency with which the animals convert feed into the desired output. For animals raised for meat (such as beef cows, pigs, chickens and fish) the output is the flesh, that is, the body mass gained by the animal, represented either in the final mass of the animal or the mass of the dressed output.

The term "minimum inhibitory concentration" or "MIC" as used herein refers to the lowest concentration of a compound or composition which prevents measurable growth of a bacterium.

The term "minimum bactericidal concentration" or "MBC" as used herein refers to the lowest concentration of a compound or composition which results in microbial death as defined by the inability to re-culture bacteria.

The term "colony-forming unit" (CFU) refers to a unit that is used to estimate the number of viable bacteria or fungal cells in a sample, that can be for example a cell culture, a feed additive or a feed composition. Viable is defined as the ability to multiply via binary fission under the controlled conditions.

In a first aspect, the invention provides a composition for use in the prevention, suppression and / or treatment of piscirickettsiosis, preferably, in aquatic poikilotherms, characterized in that said composition comprises one or more medium-chain fatty acids (MCFAs) or derivatives thereof. MCFA(s) are fatty acids with a medium-chain length, wherein the fatty acids may be saturated or unsaturated. In the present invention, the longest continuous chain of an MCFA consists of 5 (C5) to 12 (C12) carbon atoms. The fatty acids that can be used in this invention include both fatty acids with an even and an odd number of carbon atoms. Preferably, the composition of the invention comprises one or more MCFAs chosen from C5 to C12 or derivatives thereof. The term MCFA also refers to medium-chain fatty acids that are chemically modified, and to medium-chain fatty acids that are provided with side-chains, such as, without limitation, one or more alkyl groups, preferably C1-C10 alkyl groups. MCFA derivatives include MCFAs of which the carboxyl group is reversibly converted to a different group such as but not limited to an amide, a salt, an ester or a glyceride such as, for example, a mono-, di- or tri-glyceride.

Piscirickettsiosis is the main health challenge in the cold water aquaculture sector, affecting both productivity and profitability, resulting in a huge economic impact of this disease on the aquaculture sector. The inventors have found that a composition comprising at least one MCFA or MCFA derivative is surprisingly effective for the prevention, suppression, and/or treatment of this disease, thus allowing to significantly increase both the productivity and profitability of aquaculture.

Furthermore, piscirickettsiosis is a cruel and nasty disease whose impact on animal well-being should not be underestimated. Fish suffering from the disease show, amongst other symptoms, extreme weight loss, skin lesions that progress to ulcers on flanks and head, a grey, swollen spleen and kidney diffuse hemorrhaging in the liver and diverse lesions resulting from granulomas and areas of tissue necrosis. Unfortunately, piscirickettsiosis infection in many cases eventually leads to the death of the animal. The current invention thus provides a composition that can make a considerable contribution to the reduction of animal suffering and the improvement of animal welfare.

In order to reduce the occurrence of diseases in cold water aquaculture systems, antibiotics are often administered to the animals. This strategy has proven largely unsuccessful in preventing or stopping piscirickettsiosis disease outbreaks. In addition, the problem of resistance development of pathogenic organisms and the transfer of the antibiotic substances into animal-derived products makes it very desirable to reduce the use of antibiotics. Therefore, the current invention provides an efficient alternative for the administration of antibiotics and thus helps to prevent the upcoming spread of drug resistant pathogens. Finally, the current invention also has the advantage that the need for administering antibiotics to the animals is decreased.

The inventors have found that supplying specific MCFA in the range of C5-C12, their derivatives and / or mixtures thereof to piscirickettsiosis causing bacteria inhibits their further growth and the bacteria are eventually killed by the administered MCFA. In one embodiment, the composition comprises one or more MCFAs selected from the group comprising caproic acid (C6), caprylic acid (C8), capric acid (C10), lauric acid (C12) and derivatives thereof. The inventors have found that these MCFAs and derivatives thereof are especially efficient against piscirickettsiosis causing bacteria. In the present invention, preferably use is made of a composition comprising a mixture of specific different MCFAs, the individual MCFAs containing a different number of carbon atoms. The inventors have found that a composition comprising at least caprylic acid (C8), capric acid (C10) and lauric acid (C12) or a respective derivative is remarkably effective to treat, prevent and / or suppress piscirickettsiosis. Preferably, the composition of the invention comprises:
- Between 30% and 70% caprylic acid (C8) or a derivative thereof;
- Between 15% and 55% capric acid (C10) or a derivative thereof; and
- Between 1% and 35% lauric acid (C12) or a derivative thereof based on the total weight of the composition.

The inventors have found that a composition comprising a mixture of MCFAs or MCFA derivatives as described above shows optimal antimicrobial properties towards the bacteria responsible for causing the piscirickettsiosis disease.

Due to this outstanding effectiveness on prevention, suppression and/or treatment of piscirickettsiosis, smaller doses of the composition according to the invention need to be used to obtain the desired results on animal health and the concomitant positive effects on aquaculture productivity. Therefore, the current invention leads to higher revenues for the farmers while reducing the costs of disease prevention, treatment and/or suppression.

In a further embodiment, the total amount of MCFAs and/or MCFA derivatives is comprised between 1% and 100% based on the total weight of the composition, preferably between 5% and 90%, more preferably between 15% and 80%, even more preferably between 30% and 80%, based on the total weight of the composition. The inventors have found that within the ranges mentioned above, the efficiency of the composition for use in the prevention, treatment and / or suppression of piscirickettsiosis is further increased.

The species Piscirickettsia salmonis was the first pathogen identified that causes piscirickettsiosis. The disease itself, as well as the pathogens responsible for it have, however, not been characterized well yet. Unfortunately, P. salmonis is not the only pathogen that causes piscirickettsiosis, and similar bacteria are an emerging thread to fish production. The inventors have found that bacteria which are similar to P. salmonis are also sensitive to the effects of the composition according to the invention. Accordingly, in another further embodiment, the composition is suited for use in the treatment, prevention and/or suppression of the piscirickettsiosis disease when caused by at least one obligate intracellular parasitic species, preferably when it is caused by at least one species belonging to the family of Piscirickettsiacea and / or to the order of Rickettsiales, even more preferably by at least one species selected from the group comprising P. salmonis, Piscirickettsia-like organisms (PLOs) and fish Rickettsia-like organisms (RLOs). The composition does not target a species-specific property of the pathogen, but rather a disease-specific property of several related pathogen species. The composition therefore provides a broad spectrum efficiency for use in the treatment, suppression and / or prevention of piscirickettsiosis in aquatic poikilotherms.

The composition of the invention can be conveniently used in the treatment, suppression and / or prevention of piscirickettsiosis in aquatic poikilotherms, as it can be administered orally and / or topically to the animals.

Topical administration of the composition to aquatic poikilotherms is obtained, in a further embodiment, by dissolving the composition in the swimming water of the animals. Preferably, the volume of swimming water of the animals is first reduced to limit the total amount of composition to be used. Topical administration by providing the composition directly to the swimming water of the animals offers the opportunity to treat a large number of animals simultaneously. In addition, the animals can thereafter be removed from the swimming water comprising the composition and a next group of animals can be treated in the same swimming water, thus further reducing the quantity of the composition needed per animal. In a further embodiment the composition for topical administration to the animals further comprises one or more surfactants. Without wishing to be bound by theory, surfactants are thought to improve contact between the composition and the body surface of the animal, thus optimizing the topical administration of the composition. Surfactants are selected from the group comprising non-ionic, cationic or anionic surfactants such as, but not limited to, coconut alkylbenzyl dimethylammonium chloride, dioctyl dimethylammonium chloride, tributyl tetradecylphosphonium chloride, lauryl dimethyl amine oxide, cocoamidopropyl betaine, lauramidopropyl betaine, cocamide propyl ammonium hydroxide, alkyl benzene sulphonic acids, alkyl sulphonates, carboxylic acid ethers and ethoxylated and propoxylated fatty alcohols.

Alternatively, the inventors have found that oral ingestion of the composition by the animals results in efficient treatment, suppression and/or prevention of piscirickettsiosis in those animals. Therefore, and in an alternative further embodiment, a therapeutically effective amount of the composition for use in the treatment, suppression and / or prevention of piscirickettsiosis in aquatic poikilotherms is administered orally to said aquatic poikilotherm. In a further embodiment, oral administration of the composition is attained by feeding the composition to the animals. This method of administration provides the advantage that a minimal amount of composition is used and, consequently, a minimal amount of composition is wasted or remains in the swimming water of the animals. In addition, this method is easy to perform and it does not impose the burden of large investments for novel equipment on the farmer.

In a further embodiment, the composition further comprises vitamins, minerals, trace elements or a combination thereof. Vitamins include but are not limited to Vitamin A, Vitamin D3, Vitamin E, Vitamin K3, Thiamin, Riboflavin, Panthothenic acid, Biotin, Folic acid, Vitamin B12, Niacin, Pyridoxine, Ascorbic acid, Inositol and Choline. Minerals and trace elements include but are not limited to magnesium, sodium, manganese, iron, zinc, copper, selenium, phosphorous, cobalt and iodine. The addition of these components further contributes to a complete and healthy diet of the animals and positively influences their zootechnical performance. Inclusion of these components in the composition simplifies the steps needed for feeding of the animals as these components no longer need to be added separately to the feed, resulting in an improved efficiency of the feeding process. In addition, the inventors have found that the provision of MCFAs and/or MCFA derivatives in the animals' feed results in an increase in feed palatability, which in turn leads to animals that eat more, grow bigger, faster and / or healthier, which eventually leads to an increase in the profitability of aquaculture.

Although piscirickettsiosis was first observed in a Coho salmon (Oncorhynchus kisutch), the disease is now known to affect a wide range of aquatic poikilotherms including, but not limited to salmonids, white seabass (Atactoscion noblis), black seabass (Dicentrarchus sp.), tilapia (Oreochromis, Tilapia and Sarotherodon spp.) and blue-eyed plecostomus (Panaque suttoni). The term salmonid refers to fish species belonging to the family of Salmonidae including, but are not limited to, salmons, trouts, chars, freshwater whitefishes and graylings. Salmons include, but are not limited to, Atlantic salmon species such as Atlantic salmon (Salmo salar) and Pacific salmon species such as Chinook salmon (Oncorhynchus tshawytscha), Chum salmon (O. keta), Coho salmon (O. kisutch), Masu salmon (O. masou), Pink salmon (O. gorbuscha) and Sockeye salmon (O. nerka).

Because salmonids constitute the most important group of fish in aquaculture, treatment, suppression and / or prevention of piscirickettsiosis in this group of species has a prominent positive effect on the productivity and profitability of the aquaculture section. Accordingly, in a further embodiment the composition of the invention is used in the treatment, suppression and / or prevention of piscirickettsiosis in salmonids, more in particular of salmons, trouts, freshwater whitefishes and graylings.

In a further embodiment, the composition according to the invention as described herein, is formulated as a liquid or as a solid form. The term "solid form" means a powder, a granule or a pellet in particular. The term "liquid form", in particular, means a solution in water or means a solution in oil and includes a viscous paste and a non-viscous solution. The MCFAs and / or MCFA derivatives as described above are oil-soluble and can be provided both as powder and as an oil-solution. In particular, the composition is suitable for oral administration. A fish feed or feed additive comprising the composition according to an embodiment of the invention is produced or manufactured by means known to a person skilled in the art. Preferably, the fish feed comprising the composition according to the invention is provided as a dry extruded feed pellet. This formulation allows a relatively long shelf life and also permits the packaging and storage of large amounts of feed.

Oral administration of the composition provides, in contrast to other methods of administration, the advantage that a minimal amount of composition is used and, consequently, a minimal amount of composition is wasted or remains in the swimming water of the animals. In addition oral administration is easy to perform and it does not require large investments for novel equipment. Oral administration of the composition can be performed by admixing the animal feed with the composition of the invention prior to feeding, by feeding the animal with an animal feed already comprising the composition. Accordingly, and in a third aspect, the invention provides a fish feed comprising a composition for use as described above, wherein the dosage of said MCFA in said fish feed is 100 ppm to 5000 ppm, between 100 and 3000 ppm by weight, between 100 and 1000 ppm, more preferably 100 and 500 ppm, more preferably 100 and 300 ppm, such as 225 ppm based on the total weight of said fish feed.

In another embodiment, said composition is added to the water of said aquatic animals, for instance in the tank. Said composition is added such that the MCFA concentration in the water is at a concentration of 100 and 5000 ppm; between 100 and 3000 ppm, between 100 and 1000 ppm, more preferably 100 and 500 ppm, more preferably 100 and 300 ppm, such as 225 ppm.

In a second aspect, the invention provides a method for enhancing the zootechnical performance of aquatic poikilotherms comprising the step of administering a composition comprising one or more medium-chain fatty acids (MCFAs) and / or derivatives thereof to said poikilotherm. Preferably, the total amount of MCFAs and/or MCFA derivatives is comprised between 1% and 100% based on the total weight of the composition, more preferably between 5% and 90%, even more preferably between 10% and 80%, most preferably between 10% and 50%, more preferably between 10 and 30% such as 15% based on the total weight of the composition. The inventors have found that, independent of the presence or absence of piscirickettsiosis, administration of the composition of the invention to the aquatic poikilotherms, leads to an unexpected increase in the productivity of the animals, as a consequence of their improved zootechnical performance. This positive effect can be measured, for example, based on the animals' growth or based on the nutritional value of the animals and their derived products. Evidently, other methods to measure the zootechnical performance of the animals, which are known to the person skilled in the art can also be used in combination with the invention. For instance, growth can be measured at the end of the growing period as the animal's weight or the animal's size (such as fish length for example). Alternatively, the animal's growth can be measured over a determined period of time resulting in the growth rate of the animal over time. The zootechnical performance can furthermore also be measured as the swimming capacity of the animal. Finally, farmers often evaluate the zootechnical performance of animals based on the measurement of their feed conversion ratio or FCR. The FCR describes the efficiency with which the animals convert feed into the desired output. For animals raised for meat (such as beef cows, pigs, chickens and fish) the output is the flesh, that is, the body mass gained by the animal, represented either in the final mass of the animal or the mass of the dressed output.

The method described in the current invention is applicable to all cultured aquatic poikilotherms, and is particularly well suited for salmonids. Notably, salmonids tend to have a lower FCR than other cultured aquatic poikilotherms, and thus are more efficient at converting feed into body mass. The inventors have found that members of this group of fish are especially sensitive to the effects on zootechnical performance upon oral administration of the composition, which is reflected by a significant further decrease in the FCR. Therefore, application of the method in salmonid farming results in a major increase in revenues for the farmer. Additionally, because salmonids are fish-eating fish, a further decrease in FCR also translates into a lower burden on the environment because feeding of a lower quantity of wild fish results in an unaffected salmonid yield.

In a further embodiment, the fish feed further comprises other well-known ingredients so as to provide a nutritionally balanced complete food, including, but not limited to, plant matter, e.g., flour, meal, starch or cracked or processed grain produced from a crop plant such as wheat or other cereals, alfalfa, corn, oats, potato, rice, soybeans or other legumes; cellulose in a form that may be obtained from wood pulp, grasses, plant leaves, and waste plant matter such as rice or soy bean hulls, or corn cobs; animal matter, e.g., fish or crustacean meal, oil, protein or solubles and extracts, krill, meat meal, bone meal, feather meal, blood meal, or cracklings; algal matter; yeast; bacteria; vitamins, minerals, and amino acids; organic binders or adhesives; and chelating agents and preservatives.

In another further embodiment, administration of the composition according to the invention is performed orally or by feeding with a feed according to the invention. Oral administration can be performed in any manner and according to any feeding schedule employed in fish or crustacean cultivation. Feeding rates typically vary according to abiotic factors, mainly seasonal such as temperature, and biotic, in particular the size and the developmental stage of the animal.

Fish development recognizes 5 periods that occur in the following order: embryonic period; larval period; juvenile period; adult period and senescent period. The larval period occurs once the embryo has hatched and has the ability to feed independently of the egg yolk (or mother in rare cases), organ systems develop morphologically and gain physiological function. The juvenile period is when all organ systems are fully formed and functional (except the gonads) and fish attain the appearance of miniature adults. The juvenile period lasts until the gonads become mature. Once the gonads mature the fish attain the adult period. Finally, the senescence period is attained when growth ceases and gonads cease to produce gametes.

Juvenile fish are typically fed 5-10% of their body weight per day over about 4-6 feeds per day. Larger fish are typically fed at 2-5% of their body weight per day over about 1-2 feeds per day. Juvenile crustaceans may fed up to 5-10% of their body weight over about 6 feeds per day, while larger crustaceans may be fed a minimum of about 2% of their body weight per day over about 2-3 feeds per day. The fish or crustaceans may be allowed to feed to appetite.

Accordingly, in a further embodiment of the method according to the invention, the dosage of the MCFAs and / or MCFA derivatives comprises from 100 ppm to 5000 ppm by weight, based on the total weight of said aquatic poikilotherms' feed, preferably between 100 and 5000 ppm; between 100 and 3000 ppm, between 100 and 1000 ppm, more preferably 100 and 500 ppm, more preferably 100 and 300 ppm, such as 225 ppm.

Preferably, the animals are fed at least once per day, more preferably two or more times per day such as, for example, 2-6 or 4-6 times per day. It is preferred that any excess food be removed after the feeding period, e.g., by flushing out of a raceway system, or through removal out of the bottom of the sea-cage. Alternatively, a fish such as a catfish can be added to the fish population to consume any excess food.

In another embodiment, said composition is provided to then environment of the aquatic animals, such as the swimming water or tank. Said composition is added such that the MCFA concentration in the water is at a concentration of 100 and 5000 ppm; between 100 and 3000 ppm, between 100 and 1000 ppm, more preferably 100 and 500 ppm, more preferably 100 and 300 ppm, such as 225 ppm.

The invention is further described by the following non-limiting examples which further illustrate the invention, and are not intended to, nor should they be interpreted to, limit the scope of the invention.

### EXAMPLES

### Example 1. In vitro inhibition of Piscirickettsia salmonis growth by a composition according to an embodiment of the invention.

A naturally occurring strain of P. salmonis (field strain PM-15972) was used. Bacterial growth was evaluated based on M49-A of the CLSI/NCCLS standards.

Bacterial growth medium (minimum essential medium (according Eagle) with 2 mM L-glutamine and Earle's BSS adjusted to contain 1.5 g/L sodium bicarbonate, 0.1 mM non-essential amino acids, 1.0 mM sodium pyruvate and 10 mM HEPES, 90%; fetal bovine serum, 10%) containing increasing concentrations of the composition and a control (bacterial growth medium lacking MCFA or MCFA derivatives) were inoculated with an equal amount of P. salmonis (PM-15972). The mixtures were incubated, and the bacterial growth was scored: positive for the presence of bacterial growth; negative for the absence of bacterial growth. The results are shown in Table 1.

**Table 1. In vitro inhibition of P. salmonis growth in the presence of MCFAs.**

| Composition concentration | P. salmonis growth |
|---|---|
| Control (0ppm) | Positive |
| 60 ppm | Negative/Positive |
| 100 ppm | Negative |
| 300 ppm | Negative |
| 1000 ppm | Negative |
| 5000 ppm | Negative |
| 120 ppm | Negative |
| 5000 ppm | Negative |

Although P. salmonis was still able to grow in the presence of 60 ppm of the composition, the minimum inhibitory concentration (MIC) was reached in vitro at 70 ppm of the composition, as no P. salmonis growth could be detected in the presence of 70 ppm or more of the composition in the medium.

### Example 2: In vitro viability of Piscirickettsia salmonis in the presence of a composition according to an embodiment of the invention.

A naturally occurring strain of P. salmonis (field strain PM-15972) was used. Bacterial survival was evaluated based on protocol BS EN 1656:2009 of the British Standards Institution (BSI).

An aqueous solution containing increasing concentrations of the composition were inoculated with an equal amount of P. salmonis (PM-15972). The mixtures were incubated at 18°C. After 3 hours the composition was neutralized by dilution and the solution was plated on P. salmonis medium. The plates were incubated for 7 to 10 days at 18°C and the viability of the bacteria was calculated based on the colony forming units (CFU) /ml. The "CFU" is a unit that is used to estimate the number of viable bacteria in a sample. "Viable" is defined as the ability to multiply via binary fission under the controlled conditions. The results are shown in table 2.

**Table 2. In vitro survival of P. salmonis in the presence of MCFAs.**

| Composition concentration | P. salmonis viability^{a} | P. salmonis kill off ^{b} |
|---|---|---|
| Control | 5.53 x 10⁸ | 0% |
| 60 ppm | 1.20 x 10⁵ | 99.9% |
| 70 ppm | 5.07 x 10⁴ | 99.99% |
| 80 ppm | 2.30 x 10³ | 99.999% |
| 90 ppm | 0 | 100% |
| 100 ppm | 0 | 100% |
| 110 ppm | 0 | 100% |
| 120 ppm | 0 | 100% |
| 3000 ppm | 0 | 100% |

| | | |
|---|---|---|
| ^{a} P. salmonis viability is expressed as CFU/ml ^{b} P. salmonis kill off is expressed as the reduction in P. salmonis viability (compared to the control sample). | | |

From these results it can be concluded that the minimum bactericidal concentration or MBC, the lowest concentration of the composition which results in efficient P. salmonis kill off is 80 ppm according to the standard protocol (99,999%). Furthermore, this test shows that at a concentration of 90 ppm or more the composition was 100% efficient in reducing the viability of P. salmonis.

### Example 3: In vivo experiment on zootechnical performance / piscirickettsiosis treatment, suppression and / or prevention

Set up: fish tanks with 50 salmons infected with P. salmonis strain LF-89 (ATCC VR-1361) were treated with either 225 ppm of an MCFA composition of 40% C8, 40% C10 and 5% C12 in the water volume of the tank, or a control agent without MCFA. Results of the treatment were evaluated after 10 days of treatment:

| **MCFA treated population** | **Control population** |
|---|---|
| 2 deaths, overall healthy looking population | 25 deaths, population clearly sick |

After 15 days, the amount of deaths in the control population rose to 34, while in the MCFA treated tank no further deaths were reported.

This experiment was repeated with infected trouts. Also here the lesser deaths in the MCFA treated population was observed compared to the control population. To conclude, MCFAs can be used as an effective treatment of piscirickettsiosis.

It is supposed that the present invention is not restricted to any form of realization described previously and that some modifications can be added to the presented example without reappraisal of the appended claims. For example, the present invention has been described referring to P. salmonis, but it is clear that the invention can be applied to piscirickettsiosis caused by Piscirickettsia-like organisms (PLO) or, for instance, fish rickettsia-like organisms (RLO).

## Claims

1. A composition for use in the treatment, suppression and/or prevention of piscirickettsiosis, preferably in aquatic poikilotherms, **characterized in that** said composition comprises one or more medium-chain fatty acids (MCFAs) and/or derivative thereof, wherein the derivative is an amide, salt, ester, or glyceride formed at the carboxyl group thereof.

2. The composition for use according to the previous claim, **characterized in that** said MCFAs are chosen from the group comprising fatty acids with a longest continuous chain of 5 carbon atoms to 12 carbon atoms and derivative thereof, wherein the derivative is an amide, salt, ester, or glyceride formed at the carboxyl group thereof.

3. The composition for use according to any one of the previous claims, **characterized in that** said MCFAs are selected from the group comprising caproic acid (C6), caprylic acid (C8), capric acid (C10), lauric acid (C12), derivative thereof, wherein the derivative is an amide, salt, ester or glyceride formed at the carboxyl group thereof or a combination thereof.

4. The composition for use according to any one of the previous claims, **characterized in that** said composition comprises at least caprylic acid (C8), capric acid (C10) and lauric acid (C12) or derivative thereof, wherein the derivative is an amide, salt, ester, or glyceride formed at the carboxyl group thereof.

5. The composition for use according to claim 4, **characterized in that** said composition comprises
between 30% and 70% caprylic acid (C8) or derivative thereof, wherein the derivative is an amide, salt, ester, or glyceride formed at the carboxyl group thereof;
between 15% and 55% capric acid (C10) or derivative thereof, wherein the derivative is an amide, salt, ester, or glyceride formed at the carboxyl group thereof; and
between 1% and 35% lauric acid (C12) or derivative thereof, wherein the derivative is an amide, salt, ester or glyceride formed at the carboxyl group thereof;
based on the total weight of the composition.

6. The composition for use according to any of the previous claims, **characterized in that** the total amount of MCFAs in the composition is comprised between 1% and 100%, more preferably between 5 and 50% based on the total weight or volume of the composition.

7. The composition for use according to any one of the previous claims, **characterized in that** said piscirickettsiosis is caused by *Piscirickettsia salmonis.*

8. The composition for use according to any one of the previous claims, **characterized in that** a therapeutically effective amount of said composition is administered orally to an aquatic poikilotherm.

9. The composition for use according to any one of the previous claims, **characterized in that** said composition further comprises vitamins, minerals, trace elements or a combination thereof.

10. The composition for use according to any one of the previous claims, **characterized in that** said composition is administered as a powder, a granule, a pellet, a liquid or a paste.

11. The composition for use according to any one of the previous claims, **characterized in that** the composition is administered to an aquatic poikilotherm, wherein said aquatic poikilotherm is a salmonid.

12. The composition for use according to any one of the previous claims, **characterized in that** said composition is administered to a poikilotherm selected from the group comprising salmons, trouts, freshwater whitefishes and graylings.

13. A fish feed comprising a composition for use according to any of the previous claims 1-12, wherein the dosage of said composition in said fish feed is 100 ppm to 5000 ppm by weight, based on the total weight of said fish feed.

## Patentansprüche

1. Zusammensetzung zur Verwendung bei der Behandlung, Unterdrückung und/oder Prävention von Piscirickettsiose, vorzugsweise bei wechselwarmen Wassertieren, **dadurch gekennzeichnet, dass** die Zusammensetzung eine oder mehrere mittelkettige Fettsäuren (MCFAs) und/oder Derivate davon umfasst, wobei das Derivat ein Amid, ein Salz, ein Ester oder ein Glycerid ist, das/der an deren Carboxylgruppe gebildet ist.

2. Zusammensetzung zur Verwendung nach dem vorhergehenden Anspruch, **dadurch gekennzeichnet, dass** die MCFAs aus der Gruppe ausgewählt sind, die Fettsäuren mit einer längsten kontinuierlichen Kette von 5 Kohlenstoffatomen bis 12 Kohlenstoffatomen und Derivate davon umfasst, wobei das Derivat ein Amid, ein Salz, ein Ester oder ein Glycerid ist, das/der an deren Carboxylgruppe gebildet ist.

3. Zusammensetzung zur Verwendung nach einem der vorhergehenden Ansprüche, **dadurch gekennzeichnet, dass** die MCFAs aus der Gruppe ausgewählt sind, die Capronsäure (C6), Caprylsäure (C8), Caprinsäure (C10), Laurinsäure (C12), Derivate davon umfasst, wobei das Derivat ein Amid, ein Salz, ein Ester oder ein Glycerid ist, das/der an deren Carboxylgruppe gebildet ist oder eine Kombination davon.

4. Zusammensetzung zur Verwendung nach einem der vorhergehenden Ansprüche, **dadurch gekennzeichnet, dass** die Zusammensetzung mindestens Caprylsäure (C8), Caprinsäure (C10) und Laurinsäure (C12) oder Derivate davon umfasst, wobei das Derivat ein Amid, ein Salz, ein Ester oder ein Glycerid ist, das/der an deren Carboxylgruppe gebildet ist.

5. Zusammensetzung zur Verwendung nach Anspruch 4, **dadurch gekennzeichnet, dass** die Zusammensetzung, bezogen auf das Gesamtgewicht der Zusammensetzung, Folgendes umfasst:
zwischen 30 % und 70 % Caprylsäure (C8) oder ein Derivat davon, wobei das Derivat ein Amid, ein Salz, ein Ester oder ein Glycerid ist, das/der an deren Carboxylgruppe gebildet ist,
zwischen 15 % und 55 % Caprinsäure (C10) oder ein Derivat davon, wobei das Derivat ein Amid, ein Salz, ein Ester oder ein Glycerid ist, das/der an deren Carboxylgruppe gebildet ist,
zwischen 1 % und 35 % Laurinsäure (C12) oder ein Derivat davon, wobei das Derivat ein Amid, ein Salz, ein Ester oder ein Glycerid ist, das/der an deren Carboxylgruppe gebildet ist.

6. Zusammensetzung zur Verwendung nach einem der vorhergehenden Ansprüche, **dadurch gekennzeichnet, dass** die Gesamtmenge der MCFAs in der Zusammensetzung, bezogen auf das Gesamtgewicht oder -volumen der Zusammensetzung, zwischen 1 % und 100 %, bevorzugter zwischen 5 und 50 % beträgt.

7. Zusammensetzung zur Verwendung nach einem der vorhergehenden Ansprüche, **dadurch gekennzeichnet, dass** die Piscirickettsiose durch *Piscirickettsia salmonis* verursacht wird.

8. Zusammensetzung zur Verwendung nach einem der vorhergehenden Ansprüche, **dadurch gekennzeichnet, dass** eine therapeutisch wirksame Menge der Zusammensetzung einem wechselwarmen Wassertier oral verabreicht wird.

9. Zusammensetzung zur Verwendung nach einem der vorhergehenden Ansprüche, **dadurch gekennzeichnet, dass** die Zusammensetzung ferner Vitamine, Mineralstoffe, Spurenelemente oder eine Kombination davon umfasst.

10. Zusammensetzung zur Verwendung nach einem der vorhergehenden Ansprüche, **dadurch gekennzeichnet, dass** die Zusammensetzung als ein Pulver, ein Granulat, ein Pellet, eine Flüssigkeit oder eine Paste verabreicht wird.

11. Zusammensetzung zur Verwendung nach einem der vorhergehenden Ansprüche, **dadurch gekennzeichnet, dass** die Zusammensetzung einem wechselwarmen Wassertier verabreicht wird, wobei das wechselwarme Wassertier ein Lachsfisch ist.

12. Zusammensetzung zur Verwendung nach einem der vorhergehenden Ansprüche, **dadurch gekennzeichnet, dass** die Zusammensetzung einem wechselwarmen Tier verabreicht wird, das aus der Gruppe ausgewählt ist, die Lachsen, Forellen, Coregonen und Äschen umfasst.

13. Fischfutter, eine Zusammensetzung zur Verwendung nach einem der vorhergehenden Ansprüche 1 bis 12 umfassend, wobei die Dosierung der Zusammensetzung in dem Fischfutter, bezogen auf das Gesamtgewicht des Fischfutters, 100 ppm bis 5000 ppm nach Gewicht beträgt.

## Revendications

1. Composition destinée à être utilisée dans le traitement, la suppression et/ou la prévention de la piscirickettsiose, de préférence chez les poïkilothermes aquatiques, **caractérisée en ce que** ladite composition comprend un ou plusieurs acides gras à chaîne moyenne (AGCM) et/ou un dérivé de ceux-ci, dans laquelle le dérivé est un amide, un sel, un ester, ou un glycéride formé au niveau de leur groupe carboxyle.

2. Composition destinée à être utilisée selon la revendication précédente, **caractérisée en ce que** lesdits AGCM sont choisis dans le groupe comprenant les acides gras avec une chaîne continue la plus longue de 5 atomes de carbone à 12 atomes de carbone et des dérivés de ceux-ci, dans laquelle le dérivé est un amide, un sel, un ester, ou un glycéride formé au niveau de leur groupe carboxyle.

3. Composition destinée à être utilisée selon l'une quelconque des revendications précédentes, **caractérisée en ce que** lesdits AGCM sont choisis dans le groupe comprenant l'acide caproïque (C6), l'acide caprylique (C8), l'acide caprique (C10), l'acide laurique (C12), et des dérivés de ceux-ci, dans laquelle le dérivé est un amide, un sel, un ester, ou un glycéride formé au niveau de leur groupe carboxyle ou une combinaison de ceux-ci.

4. Composition destinée à être utilisée selon l'une quelconque des revendications précédentes, **caractérisée en ce que** ladite composition comprend au moins l'acide caprylique (C8), l'acide caprique (C10) et l'acide laurique (C12) ou un dérivé de ceux-ci, dans laquelle le dérivé est un amide, un sel, un ester, ou un glycéride formé au niveau de leur groupe carboxyle.

5. Composition destinée à être utilisée selon la revendication 4, **caractérisée en ce que** ladite composition comprend
entre 30 % et 70 % d'acide caprylique (C8) ou d'un dérivé de celui-ci, dans laquelle le dérivé est un amide, un sel, un ester, ou un glycéride formé au niveau de son groupe carboxyle ;
entre 15 % et 55 % d'acide caprique (C10) ou d'un dérivé de celui-ci, dans laquelle le dérivé est un amide, un sel, un ester, ou un glycéride formé au niveau de son groupe carboxyle ; et
entre 1 % et 35 % d'acide laurique (C12) ou d'un dérivé de celui-ci, dans laquelle le dérivé est un amide, un sel, un ester, ou un glycéride formé au niveau de son groupe carboxyle ;
par rapport au poids total de la composition.

6. Composition destinée à être utilisée selon l'une quelconque des revendications précédentes, **caractérisée en ce que** la quantité totale d'AGCM dans la composition est comprise entre 1 % et 100 %, plus préférentiellement entre 5 et 50 % par rapport au poids ou au volume total de la composition.

7. Composition destinée à être utilisée selon l'une quelconque des revendications précédentes, **caractérisée en ce que** la piscirickettsiose est causée par la *Piscirickettsia salmonis.*

8. Composition destinée à être utilisée selon l'une quelconque des revendications précédentes, **caractérisée en ce qu'**une quantité thérapeutiquement efficace de ladite composition est administrée par voie orale à un poïkilotherme aquatique.

9. Composition destinée à être utilisée selon l'une quelconque des revendications précédentes, **caractérisée en ce que** ladite composition comprend en outre des vitamines, des minéraux, des oligo-éléments ou une combinaison de ceux-ci.

10. Composition destinée à être utilisée selon l'une quelconque des revendications précédentes, **caractérisée en ce que** ladite composition est administrée sous forme d'une poudre, d'un granulé, d'une pastille, d'un liquide ou d'une pâte.

11. Composition destinée à être utilisée selon l'une quelconque des revendications précédentes, **caractérisée en ce que** la composition est administrée à un poïkilotherme aquatique, dans laquelle ledit poïkilotherme aquatique est un salmonidé.

12. Composition destinée à être utilisée selon l'une quelconque des revendications précédentes, **caractérisée en ce que** ladite composition est administrée à un poïkilotherme choisi dans le groupe comprenant les saumons, les truites, les corégones d'eau douce et les ombres.

13. Aliment pour poissons comprenant une composition destinée à être utilisée selon l'une quelconque des revendications précédentes 1 à 12, dans lequel le dosage de ladite composition dans ledit aliment pour poissons est de 100 ppm à 5000 ppm en poids, par rapport au poids total dudit aliment pour poissons.
